# EUROPEAN PATENT SPECIFICATION

(11) **EP 0 661 046 B1**
(45) Date of publication and mention of the grant of the patent: **30.09.1998**
(21) Application number: 94120552.8
(22) Date of filing: 23.12.1994
(51) Int. Cl.: A61K 31/34, A61L 15/44

(54) **Transdermal patch with isosorbide dinitrate as active ingredient**
Transdermales Pflaster mit Isosorbiddinitrat als aktivem Bestandteil
Pansement transdermique avec le dinitrate d'isosorbide comme ingrédient actif

(30) Priority: 30.12.1993 EP 93121141; 05.09.1994 EP 94113894
(43) Date of publication of application: 05.07.1995
(73) Proprietor: Rotta Research B.V., 1017 PS Amsterdam (NL)
(72) Inventor: Rovati, Lucio C., c/o Rotta Research Lab. S.p.A., I-20052 Monza (IT); Senin, Paolo, c/o Rotta Research Lab. S.p.A., I-20052 Monza (IT); Setnikar, Ivo, c/o Rotta Research Lab. S.p.A., I-20052 Monza (IT)
(74) Representative: Boeters, Hans Dietrich, Dr.

(56) References cited:
- EP-A- 0 181 970
- EP-A- 0 337 358
- EP-A- 0 531 938
- EP-A- 0 563 024
- FR-A- 2 497 457

## Description

EP-A-0 337 358 describes a percutaneous absorption type preparation comprising a drug-impermeable backing having provided thereon an adhesive layer comprising a pressure-sensitive adhesive containing a drug in an amount greater than the saturated solubility in the adhesive, wherein the excess amount of the drug greater than the saturate solubility is present in the adhesive in a crystallized state. With respect to an easy availability of drugs it has, however, always been a need to provide drugs an a dissolved state.

Further, EP-A-0 531 938 suggests a medical preparation for percutaneous absorption of a drug comprising an acrylic gel material comprising a substrate having formed on at least one side thereof a cross-linked pressure-sensitive adhesive layer containing an acrylic ester-based polymer and an organic liquid component. Medical preparations avoiding any organic liquid are, however, preferred since liquid components might cause irritations on the skin.

Further, EP-A-0 563 024 suggests a salve preparation containing isosorbide dinitrate (ISDN) as active ingredient. As regards acceptance of preparations, transdermal patches are preferred. Thus, EP-A-0 181 970 suggests a percutaneously administrable pharmaceutical preparation in the form of an adhesive tape comprising a flexible backing which is not permeable to ISDN, and an adhesive coating layer on said flexible backing, the adhesive layer comprising an adhesive material, wherein said adhesive material is a copolymer derived from 2-ethyl-hexyl acrylate and N-vinyl-2-pyrrolidone and wherein ISDN is present in said adhesive material in a concentration of at least 10 % by weight. There has, however, always been a demand to improve the capacity of transdermal patches for ISDN.

DE-B-3 200 369 describes a transdermal patch comprising an outer covering supporting a preparation containing isosorbide dinitrate (ISDN) at a concentration of 0.5 to 20 percent by weight, in a polymer having a glass transition temperature (Tg) of -70 to -10 °C, the polymer being a copolymer of an alkylacrylate such as 2-ethylhexyl acrylate, with a functional monomer copolymerizable therewith, such as acrylic acid, or a vinylester monomer copolymerizable therewith, such as vinyl acetate. The preferred concentration of the active ingredient is 2 to 15 percent by weight. This preferred concentration of the active ingredient reflects the fact that the known ability of the acrylic matrix of the transdermal patch to dissolve the active ingredient in an uncrystallized state is limited. Thus, there has still been a need to improve the solubility of ISDN in transdermal patches.

According to the invention this problem has been solved by a transdermal patch being self-adhesive at skin temperature and comprising
(a) a flexible outer covering supporting
(b) a pressure-sensitive adhesive matrix containing isosorbide dinitrate (ISDN) as active ingredient
(c) the matrix consisting of a copolymer of at least
   2-ethylhexyl acrylate in an amount of 35 to 45 parts,
   acrylic acid in an amount of 0.5 to 3 parts,
   vinyl acetate in an amount of 15 to 45 parts,
   ethyl acrylate in an amount of 5 to 44 parts of the adhesive matrix, each, including an optional hardener,
(d) wherein the content of the active ingredient is in the range of from 16 to 22 percent by weight based on the total weight of the adhesive matrix.

According to another embodiment of the invention the problem has been solved by a transdermal patch being self-adhesive at skin temperature and comprising
(a) a flexible outer covering supporting
(b) a pressure-sensitive adhesive matrix containing isosorbide dinitrate (ISDN) as active ingredient
(c) the matrix consisting of a copolymer of at least
   2-ethylhexyl acrylate in an amount of 35 to 45 parts,
   acrylic acid in an amount of 1 to 3 parts,
   vinyl acetate in an amount of 25 to 45 parts,
   ethyl acrylate in an amount of 5 to 30 parts of the adhesive matrix, each, including an optional hardener.
(d) wherein the content of the active ingredient is in the range of from 16 to 22 percent by weight based on the total weight of the adhesive matrix.

According to another embodiment of the invention the problem has been solved by a transdermal patch being self-adhesive at skin temperature and comprising
(a) a flexible outer covering supporting
(b) a pressure-sensitive adhesive matrix containing isosorbide dinitrate (ISDN) as active ingredient
(c) the matrix consisting of a copolymer of at least
   2-ethylhexyl acrylate in an amount of 35 to 45 parts,
   acrylic acid in an amount of 0.5 to 3 parts,
   vinyl acetate in an amount of 15 to 25 parts,
   ethyl acrylate in an amount of 34 to 44 parts of the adhesive matrix, each, including an optional hardener,
(d) wherein the content of the active ingredient is in the range of from 16 to 22 percent by weight based on the total weight of the adhesive matrix.

Preferably the content of residual solvents remained in the patch from its production is less than about 220 ppm.

The transdermal patch of the invention may have been produced with the use of a hardener or may have been produced without any use of a hardener. As regards useful hardeners reference can be made to the relevant art.

Besides the essential components, the matrix of the transdermal patch according to the invention may additionally contain excipients, additives, hardeners, plastifiers and other chemicals As regards these additional components and the outer covering, reference can be made to the prior art.

According to a preferred embodiment the adhesive matrix of the transdermal patch of the invention is characterized by a copolymer of
about 40 parts 2-ethylhexyl acrylate,
about 2 parts acrylic acid,
about 40 parts vinyl acetate and
about 18 parts ethyl acrylate and
a content of about 20 percent by weight isosorbide dinitrate,
   the active ingredient being carried in the adhesive matrix an uncrystallized state.

According to another preferred embodiment the adhesive matrix of the transdermal patch of the invention is characterized by a copolymer of
about 40 parts 2-ethylhexyl acrylate,
about 1 part acrylic acid,
about 20 parts vinyl acetate and
about 39 parts ethyl acrylate and
   a content of about 21 percent by weight isosorbide dinitrate, the active ingredient being carried in an uncrystallized state.

The transdermal patch according to the invention is described in detail by the following examples.

### Example 1

A copolymer resulting from 40 parts 2-ethylhexyl acrylate, 2 parts acrylic acid, 40 parts vinyl acetate and 18 parts ethyl acrylate was dissolved in a suitable organic solvent. An amount of 20 percent isosorbide dinitrate was added to the solution and the resulting mixture was gently stirred. The mixture was lined at a final thickness of 100 m on the surface of a polyethylene sheet for the production of a transdermal patch.

After a storage of 10 months at room temperature, no crystallization of the active ingredient could be observed.

### Examples 2 to 4 and Comparative Examples 1 to 3

Six formulations of acrylic copolymers were prepared according to the compositions shown in Tables 1 to 3.

**Table 1**

| Percent composition of the matrix | | | | | | |
|---|---|---|---|---|---|---|
| Example Sample | Ex. 2 63301 | Comp. Ex. 1 63302 | Comp. Ex. 2 63303 | Comp. Ex. 3 73071 | Ex. 3 73072 | Ex. 4 73073 |
| 2-Ethylhexyl acrylate | 31,20 | 72,54 | 57,72 | 44,00 | 31,20 | 31,20 |
| Ethoxy ethyl acrylate | | | | 12,00 | | |
| Ethyl acrylate | 14,04 | | | | 7,80 | 19,50 |
| Acrylic acid | 1,56 | 5,46 | 4,68 | | 1,56 | 1,56 |
| Vinyl acetate | 31,20 | | 15,60 | 24,00 | 37,44 | 25,74 |
| ISDN | 20,00 | 20,00 | 20,00 | 20,00 | 20,00 | 20,00 |
| Hardener | 2,00 | 2,00 | 2,00 | | 2,00 | 2,00 |
| sum | 100,00 | 100,00 | 100,00 | 100,00 | 100,00 | 100,00 |

**Table 2**

| Percent composition of the matrix without hardener | | | | | | |
|---|---|---|---|---|---|---|
| Example Sample | Ex. 2 63301 | Comp. Ex. 1 63302 | Comp. Ex. 2 63303 | Comp. Ex. 3 73071 | Ex. 3 73072 | Ex. 4 73073 |
| 2-Ethylhexyl acrylate | 32,00 | 74,40 | 59,20 | 44,00 | 32,00 | 32,00 |
| Ethoxy ethyl acrylate | | | | 12,00 | | |
| Ethyl acrylate | 14,40 | | | | 8,00 | 20,00 |
| Acrylic acid | 1,60 | 5,60 | 4,80 | | 1,60 | 1,60 |
| Vinyl acetate | 32,00 | | 16,00 | 24,00 | 38,40 | 26,40 |
| ISDN | 20,00 | 20,00 | 20,00 | 20,00 | 20,00 | 20,00 |
| sum | 100,00 | 100,00 | 100,00 | 100,00 | 100,00 | 100,00 |

**Table 3**

| Percent composition of the matrix without ISDN and without hardener | | | | | | |
|---|---|---|---|---|---|---|
| Example Sample | Ex. 2 63301 | Comp. Ex. 1 63302 | Comp. Ex. 2 63303 | Comp. Ex. 3 73071 | Ex. 3 73072 | Ex. 4 73073 |
| 2-Ethylhexyl acrylate | 40,00 | 93,00 | 74,00 | 55,00 | 40,00 | 40,00 |
| Ethoxy ethyl acrylate | | | | 15,00 | | |
| Ethyl acrylate | 18,00 | | | | 10,00 | 25,00 |
| Acrylic acid | 2,00 | 7,00 | 6,00 | | 2,00 | 2,00 |
| Vinyl acetate | 40,00 | | 20,00 | 30,00 | 48,00 | 33,00 |
| Sum | 100,00 | 100,00 | 100,00 | 100,00 | 100,00 | 100,00 |

The copolymers and ISDN were mechanically mixed with a mixer, lined on a polyethylene foil, dried at 80 °C for 10 minutes, protected with a silicone-coated paper used as protective liner, cut into appropriate sizes, and enclosed in aluminum sachets.

The following tests were performed with these samples after storage at 5 °C and 40 °C.
1) Test for crystallization of ISDN in the matrix after storage at 5 °C and at 40 °C by visual inspection.
2) Adhesion peeling test after storage at 5 °C and 40 °C
3) Permeability test through the skin of the rat after storage at 5 °C.

The results are reported in Tables 4, 5 and 6

**Table 4**

| Stability test for crystallization of ISDN | | | | | | | |
|---|---|---|---|---|---|---|---|
| Storage time | Storage temper. | Ex. 2 63301 | ComEx.1 63302 | ComEx. 2 63303 | ComEx. 3 73071 | Ex. 3 73072 | Ex. 4 73073 |
| 0 | | None | (1) | None | None | None | None |
| 1 Month | 5 °C | None | (1) | (2) | None | None | None |
| | 40 °C | None | (1) | None | None | None | None |
| 2 Months | 5 °C | None | (1) | (2) | None | None | None |
| | 40 °C | None | (1) | (2) | None | None | None |
| 2 Months | 5 °C | None | (1) | (2) | None | None | None |
| | 40 °C | None | (1) | (2) | None | None | None |
| Notes: (1) Crystallization (2) Severe crystallization | | | | | | | |

**Table 5**

| Adhesion peeling test (g/12 mm) | | | | | | | |
|---|---|---|---|---|---|---|---|
| Storage time | Storage temper. | Ex. 2 63301 | ComEx.1 63302 | ComEx. 2 63303 | ComEx. 3 73071 | Ex. 3 73072 | Ex. 4 73073 |
| 0 | | 557 | 424 | 388 | (2) | 222 | 412 |
| 1 Month | 5 °C | 607 | 461 | (1) | (2) | 399 | 385 |
| | 40 °C | 504 | 439 | 379 | (2) | 421 | 404 |
| 2 Months | 5 °C | 578 | 435 | (1) | (2) | 114 | 376 |
| | 40 °C | 535 | 415 | 370 | (2) | 187 | 411 |
| 2 Months | 5 °C | 543 | 478 | (1) | (2) | 103 | 353 |
| | 40 °C | 417 | 416 | 384 | (2) | 93 | 399 |
| Notes: (1) Poor adhesion due to crystallization of ISDN on the surface of the matrix (2) Loss of any adhesion property of the matrix | | | | | | | |

**Table 6**

| Permeability through the rat skin ( g/2.27 cm²) after storage at 5 °C | | | | | | | |
|---|---|---|---|---|---|---|---|
| Storage time | Hours | Ex. 2 63301 | ComEx.1 63302 | ComEx. 2 63303 | ComEx. 3 73071 | Ex. 3 73072 | Ex. 4 73073 |
| 0 | 2 | 103 | 163 | 111 | 140 | 151 | 104 |
| | 4 | 188 | 288 | 237 | 311 | 329 | 250 |
| | 8 | 364 | 561 | 488 | 311 | 329 | 250 |
| | 24 | 984 | 1434 | 1347 | 1453 | 1405 | 1312 |
| 1 Month | 2 | 112 | 110 | 99 | | | |
| | 4 | 251 | 254 | 237 | | | |
| | 8 | 476 | 494 | 480 | | | |
| | 24 | 1329 | 1477 | 1509 | | | |
| 2 Months | 2 | 140 | 160 | 138 | 97 | 102 | 112 |
| | 4 | 331 | 325 | 249 | 213 | 213 | 235 |
| | 8 | 543 | 538 | 485 | 398 | 385 | 405 |
| | 24 | 1319 | 1227 | 1329 | 983 | 905 | 969 |
| 3 Months | 2 | 135 | 129 | 83 | 65 | 79 | 111 |
| | 4 | 304 | 288 | 173 | 148 | 177 | 236 |
| | 8 | 540 | 544 | 378 | 250 | 289 | 369 |
| | 21 | 1211 | 1467 | 1333 | 773 | 797 | 953 |

The phenomenon of crystallization of ISDN in the matrix was found in sample 63 302 (Comp. Ex. 1) at 5 °C and 40 °C, immediately and after 3 months storage.

The adhesive test could not be performed on sample 63 302 (Comp. Ex. 2) at 5 °C, and on sample 73071 (Comp. Ex. 3) at 5 °C and at 40 °C, because of loss of any cohesive property in the matrix.

In a stability test for the characteristics of the adhesive matrix, it was demonstrated that samples 63302 and 73073 (Comp. Ex. 1 and Ex. 4) had the best adhesive stability, followed by sample 63303 (Comp. Ex. 2) at 40 °C, and 63301 and 73072 (Ex. 2 and 3). These data show that the stability of the polymer increases as the proportion of the vinyl acetate decreases.

In a permeability test in vitro on rat skin, it was noted that crystallization of ISDN in the adhesive matrix had not influenced the permeability in this test, because all samples showed almost the same pattern of permeability, even though samples 63302 and 63303 (Comp. Ex. 1 and 2) showed crystallized ISDN in the matrix.

Judging from the above-mentioned data, it was concluded that globally sample 63301 (Ex. 2) had the best characteristics.

### Example 5

A copolymer of 40 parts 2-ethylhexyl acrylate, 1 part acrylic acid, 20 parts vinyl acetate and 39 parts ethyl acrylate was melted. An amount of 21 percent isosorbide dinitrate was added to the melt and the resulting mixture was gently stirred. The mixture was deposited at a thickness of 100 m on the surface of a polyethylene sheet for the production of a transdermal patch.

After a storage of 10 months at room temperature, no crystallization of the active ingredient could be observed.

## Claims

1. A transdermal patch being self-adhesive at skin temperature and comprising
(a) a flexible outer covering supporting
(b) a pressure-sensitive adhesive matrix containing isosorbide dinitrate (ISDN) as active ingredient
(c) the matrix consisting of a copolymer of at least 2-ethylhexyl acrylate in an amount of 35 to 45 parts, acrylic acid in an amount of 0.5 to 3 parts, vinyl acetate in an amount of 15 to 45 parts, ethyl acrylate in an amount of 5 to 44 parts of the adhesive matrix, each, including an optional hardener,
(d) wherein the content of the active ingredient is in the range of from 16 to 22 percent by weight based on the total weight of the adhesive matrix.

2. A transdermal patch according to claim 1, **characterized** by a copolymer of at least 2-ethylhexyl acrylate in an amount of 35 to 45 parts,
acrylic acid in an amount of 1 to 3 parts,
vinyl acetate in an amount of 25 to 45 parts,
ethyl acrylate in an amount of 5 to 30 parts of the adhesive matrix, each, including an optional hardener.

3. A transdermal patch according to claim 1, **characterized** by a copolymer of at least 2-ethylhexyl acrylate in an amount of 35 to 45 parts,
acrylic acid in an amount of 0.5 to 3 parts,
vinyl acetate in an amount of 15 to 25 parts,
ethyl acrylate in an amount of 34 to 44 parts of the adhesive matrix, each, including an optional hardener.

4. A transdermal patch according to claim 2, **characterized** by a copolymer of
about 40 parts 2-ethylhexyl acrylate,
about 2 parts acrylic acid,
about 40 parts vinyl acetate and
about 18 parts ethyl acrylate and
a content of about 20 percent by weight isosorbide dinitrate, the active ingredient being carried in the adhesive matrix an uncrystallized state.

5. A transdermal patch according to claim 3, **characterized** by a copolymer of
about 40 parts 2-ethylhexyl acrylate,
about 1 part acrylic acid,
about 20 parts vinyl acetate and
about 39 parts ethyl acrylate and
a content of about 21 percent by weight isosorbide dinitrate, the active ingredient being carried in the adhesive matrix an uncrystallized state.

## Patentansprüche

1. Perkutanes Pflaster, das bei der Temperatur der Haut selbstklebend ist und umfaßt:
(a) eine flexible äußere Bedeckung, die
(b) eine druckempfindliche Haftmatrix trägert, die Isosorbiddinitrat (ISDN) als Wirkstoff enthält, wobei
(c) die Matrix aus einem Copolymer aus zumindest 2-Ethylhexylacrylat in einer Menge von 35 bis 45 Teilen, Acrylsäure in einer Menge von 0,5 bis 3 Teilen, Vinylacetat in einer Menge von 15 bis 45 Teilen, Ethylacrylat in einer Menge von 5 bis 44 Teilen, jeweils bezogen auf die Haftmatrix, besteht, und gegebenenfalls einen Härter enthält,
(d) worin der Gehalt des Wirkstoffs im Bereich von 16 bis 22 Gew.-%, bezogen auf das Gesamtgewicht der Haftmatrix, liegt.

2. Perkutanes Pflaster nach Anspruch 1, gekennzeichnet durch ein Copolmer zumindest aus 2-Ethylhexylacrylat in einer Menge von 35 bis 45 Teilen,
Acrylsäure in einer Menge von 1 bis 3 Teilen,
Vinylacetat in einer Menge von 25 bis 45 Teilen,
Ethylacrylat in einer Menge von 5 bis 30 Teilen, jeweils bezogen auf die Haftmatrix, das gegebenenfalls einen Härter enthält.

3. Perkutanes Pflaster nach Anspruch 1, gekennzeichnet durch ein Copolmer zumindest aus 2-Ethylhexylacrylat in einer Menge von 35 bis 45 Teilen,
Acrylsäure in einer Menge von 0.5 bis 3 Teilen,
Vinylacetat in einer Menge von 15 bis 25 Teilen, Ethylacrylat in einer Menge von 34 bis 44 Teilen, jeweils bezogen auf die Haftmatrix, das gegebenenfalls einen Härter enthält.

4. Perkutanes Pflaster nach Anspruch 2, gekennzeichnet durch ein Copolmer aus
etwa 40 Teilen 2-Ethylhexylacrylat,
etwa 2 Teilen Acrylsäure,
etwa 40 Teilen Vinylacetat, und
etwa 18 Teilen Ethylacrylat,
und einen Gehalt von etwa 20 Gew.-% Isosorbiddinitrat, wobei der Wirkstoff in der Haftmatrix in einem nicht kristallisierten Zustand enthalten ist.

5. Perkutanes Pflaster nach Anspruch 3, gekennzeichnet durch ein Copolmer aus
etwa 40 Teilen 2-Ethylhexylacrylat,
etwa 1 Teil Acrylsäure,
etwa 20 Teilen Vinylacetat, und
etwa 39 Teilen Ethylacrylat,
und einen Gehalt von etwa 21 Gew.-% Isosorbiddinitrat, wobei der Wirkstoff in der Haftmatrix in einem nicht kristallisierten Zustand enthalten ist.

## Revendications

1. Timbre transdermique, auto-adhésif à la température de la peau, qui comporte
a) une pièce de couverture extérieure flexible, qui supporte
b) une matrice adhésive sensible à la pression, qui contient du dinitrate d'isosorbide (ISDN) comme ingrédient actif,
c) cette matrice consistant en un copolymère constitué, au moins, d'acrylate de 2-éthyl-hexyle à raison de 35 à 45 parties, d'acide acrylique à raison de 0,5 à 3 parties, d'acétate de vinyle à raison de 15 à 45 parties, et d'acrylate d'éthyle à raison de 5 à 44 parties, en parties de la matrice adhésive pour chacun des constituants, y compris un éventuel durcisseur, et
d) la teneur en ingrédient actif se situant dans l'intervalle allant de 16 à 22 % en poids, par rapport au poids total de la matrice adhésive.

2. Timbre transdermique conforme à la revendication 1, caractérisé en ce qu'il y a un copolymère constitué, au moins,
d'acrylate de 2-éthyl-hexyle à raison de 35 à 45 parties,
d'acide acrylique à raison de 1 à 3 parties,
d'acétate de vinyle à raison de 25 à 45 parties, et
d'acrylate d'éthyle à raison de 5 à 30 parties,
en parties de la matrice adhésive pour chacun des constituants, y compris un éventuel durcisseur.

3. Timbre transdermique conforme à la revendication 1, caractérisé en ce qu'il y a un copolymère constitué, au moins,
d'acrylate de 2-éthyl-hexyle à raison de 35 à 45 parties,
d'acide acrylique à raison de 0,5 à 3 parties,
d'acétate de vinyle à raison de 15 à 25 parties, et
d'acrylate d'éthyle à raison de 34 à 44 parties,
en parties de la matrice adhésive pour chacun des constituants, y compris un éventuel durcisseur.

4. Timbre transdermique conforme à la revendication 2, caractérisé en ce qu'il y a un copolymère constitué
d'environ 40 parties d'acrylate de 2-éthyl-hexyle,
d'environ 2 parties d'acide acrylique,
d'environ 40 parties d'acétate de vinyle, et
d'environ 18 parties d'acrylate d'éthyle,
et en ce qu'il y a une teneur d'environ 20 % en poids de dinitrate d'isosorbide, l'ingrédient actif se trouvant au sein de la matrice adhésive, dans un état non cristallisé.

5. Timbre transdermique conforme à la revendication 3, caractérisé en ce qu'il y a un copolymère constitué
d'environ 40 parties d'acrylate de 2-éthyl-hexyle,
d'environ 1 partie d'acide acrylique,
d'environ 20 parties d'acétate de vinyle, et
d'environ 39 parties d'acrylate d'éthyle,
et en ce qu'il y a une teneur d'environ 21 % en poids de dinitrate d'isosorbide, l'ingrédient actif se trouvant au sein de la matrice adhésive, dans un état non cristallisé.
